# EUROPÄISCHE PATENTSCHRIFT

(11) **EP 3 402 545 B1**
(45) Veröffentlichungstag und Bekanntmachung des Hinweises auf die Patenterteilung: **01.04.2020**
(21) Anmeldenummer: 18704227.0
(22) Anmeldetag: 08.02.2018
(51) Int. Cl.: A61M 1/10, A61M 1/12

(54) **KATHETERPUMPE MIT EINEM PUMPENKOPF ZUM EINSETZEN IN DAS ARTERIELLE GEFÄSSSYSTEM**
CATHETER PUMP WITH PUMP HEAD FOR INTRODUCTION IN THE ARTERIAL VESSEL SYSTEM
POMPE A CATHETER POUR INTRODUCTION DANS LE SYSTEME DE VAISSEAUX ARTERIELS

(30) Priorität: 13.02.2017 DE 102017102823
(43) Veröffentlichungstag der Anmeldung: 21.11.2018
(73) Patentinhaber: Cardiobridge GmbH, 72379 Hechingen (DE)
(72) Erfinder: EPPLE, Klaus, 72414 Rangendingen (DE)
(74) Vertreter: DREISS Patentanwälte PartG mbB
(86) Internationale Anmeldenummer: PCT/EP2018/053122
(87) Internationale Veröffentlichungsnummer: WO 2018/146170

(56) Entgegenhaltungen:
- EP-B1- 0 768 900
- EP-B1- 2 308 422
- DE-A1-102013 008 159

## Beschreibung

Die Erfindung betrifft eine Katheterpumpe mit einem Pumpenkopf zum Einsetzen in das arterielle Gefäßsystem wie zum Beispiel die Aorta oder das Herz, wobei der Pumpenkopf einen Rotor mit Propellern aufweist, die aus einer eingeklappten Einführlage, in der der Pumpenkopf in die das arterielle Gefäßsystem einführbar ist, in eine ausgeklappte Betriebslage, in der der Rotor in Rotation versetzbar ist, verlagerbar sind, und einen die Propeller umgebenden Käfig aufweist, wobei der Käfig eine distale und eine proximale Hülse sowie zwischen den Hülsen verlaufende Filamente aufweist. Beim oder vor dem Ausklappen der Propeller werden die Hülsen so aufeinander zu bewegt werden, dass die zwischen den Hülsen liegenden Bereiche der Filamente nach radial außen expandieren, um einen für die ausgeklappten Propeller umgebenden Raum zu bilden.

Derartige Katheterpumpen sind beispielsweise aus der EP 0 768 900 B1 oder der DE102013008159A1 oder der EP 2 308 422 B1 bekannt. Als rotierendes Förderelement kann beispielsweise, wie in der EP 768 900 B1 beschrieben, ein Rotor mit ausklappbaren Propellern Verwendung finden. Ebenso ist denkbar, dass anders ausgebildete Förderelemente, wie beispielsweise helixartig ausgebildete Wendel, Verwendung finden können.

Katheterpumpen werden als temporäres Kreislaufunterstützungssystem in insbesondere die Aorta von Patienten eingesetzt, insbesondere dann, wenn das natürliche Herz nicht in der Lage ist, den Körper mit ausreichend mit Sauerstoff versetztem Blut zu versorgen. Das Förderelement und die Rotorwelle werden dabei mit vergleichsweise hohen Drehzahlen im Bereich von 7000 bis 15000 Drehungen pro Minute betrieben. Der Pumpenkopf der Katheterpumpe kann insbesondere nach einer Operation mehrere Tage in der Aorta verbleiben.

Der vorliegenden Erfindung liegt die Aufgabe zugrunde, die Hülsen des Käfigs sicher am Katheter zu anzuordnen, so dass ein sicheres Expandieren möglich ist.

Diese Aufgabe wird durch eine Katheterpumpe mit den Merkmalen des Patentanspruchs 1 gelöst. Die Erfindung sieht folglich vor, dass im Bereich der distalen und/oder der proximalen Hülse ein mit der Hülse in axialer Richtung gekoppeltes Trageteil vorgesehen ist, wobei das Trageteil eine Umfangsnut aufweist, in der ein in axialer Richtung gefangenes Ringelelement vorgesehen ist, und wobei die Hülse wenigstens eine Ausnehmung aufweist, über welche die Hülse mit dem Ringelement verschweißt ist.

Das proximale Trageteil ist dabei vorzugsweise mit dem Außenkatheter in axialer Richtung bewegungsgekoppelt. Das distale Trageteil ist dabei vorzugsweise in axialer Richtung mit dem Innenkatheter bewegungsgekoppelt. Durch eine Relativbewegung zwischen dem Innenkatheter und dem Außenkatheter kann dadurch der Käfig, und letztlich auch das Förderelement, expandiert bzw. kollabiert werden.

Das Verschweißen der jeweiligen Hülse mit dem zugehörigen Ringelement hat den Vorteil, dass die Hülse und das Ringelement aus einem anderen Material sein können als das jeweilige Trageteil. Das Ringelement ist dabei in einer am Trageteil vorgesehenen Umfangsnut in axialer Richtung formschlüssig gefangen angeordnet, so dass es in axialer Richtung vorzugsweise ohne oder nur mit geringem Spiel relativ zum Trageteil bewegbar ist. Hierdurch kann sichergestellt werden, dass axiale Kräfte zum Aufeinander zu oder voneinander weg bewegen der Hülsen sicher vom Trageteil auf das Ringelement und damit auf die Hülse übertragen werden. Die axiale Kopplung der Hülsen an den entsprechenden Trageteilen ist wichtig, um ein funktionssicheres Aus- und Einklappen der Propeller des Rotors zu gewährleisten. Das axiale Verschieben des proximalen Trageteils erfolgt dabei letztlich durch axiales Bewegen des Außenkatheters relativ zum Innenkatheter.

Insgesamt kann hierdurch eine sichere Anbindung der Hülsen über die Ringelemente an den jeweiligen Trageteilen gewährleistet werden.

Das Ringelement kann insbesondere als geschlitztes Bauteil ausgebildet sein, wodurch sich insbesondere eine Montageerleichterung und ein Toleranzausgleich ergeben.

Der Käfig samt Hülsen und das Ringelement sind vorzugsweise aus dem gleichen Material und insbesondere aus einer Formgedächtnislegierung. Dadurch kann ein Verschweißen der Bauteile auf vergleichsweise einfache Art erfolgen. Das Trageteil ist vorzugsweise aus einem zum Käfig unterschiedlichen Material und besteht vorzugsweise aus einer Edelstahllegierung.

Bei der wenigstens einen an der Hülse vorgesehenen Ausnehmung handelt es sich insbesondere um ein Rundloch oder um ein Langloch. Die Löcher können beispielsweise gebohrt, gestanzt oder laserausgeschnitten hergestellt sein. Die Ausnehmungen haben dabei eine Größe, so dass mittels einer geeigneten Schweißeinrichtung die Hülse mit dem Ringelement verschweißt werden kann.

Vorteilhafterweise ist der Käfig samt Hülsen einstückig ausgebildet und aus einer Formgedächtnislegierung. Besonders vorteilhaft hat sich eine Formgedächtnislegierung in Form einer Nickel-Titan-Legierung herausgestellt. Aufgrund der guten Verformbarkeit und der guten Korrosionsfestigkeit eignet sich die Legierung insbesondere für den Käfig. Das oder die Ringelemente können, wie bereits erwähnt, aus der gleichen Legierung sein.

Vorteilhafterweise weist die jeweilige Hülse, und insbesondere beide Hülsen, mehrere Ausnehmungen auf. Insbesondere können die Ausnehmungen auf einer umlaufenden Kreisbahn äquidistant zueinander angeordnet sein. Denkbar ist auch, dass mehrere parallel zueinander verlaufende Kreisbahnen mit entsprechenden Ausnehmungen vorgesehen sind.

Die am freien Ende der Katheterpumpe vorgesehene distale Hülse des Käfigs ist vorzugsweise über ein zugehöriges Ringelement an einem distalen Trageteil angeordnet, wobei das distale Trageteil eine Drehlageraufnahme für das distale Ende des Rotors bildet. Auf seiner radial außenliegenden Seite sieht das Trageteil die Umfangsnut für das Ringelement vor. Auf seiner radial inneren Seite kann das Trageteil die Drehlageraufnahme für den Rotor vorsehen.

Die proximale Hülse ist vorzugsweise an einem proximalen Trageteil angeordnet, wobei das proximale Trageteil eine Schiebelageraufnahme aufweist, die eine axiale Bewegung zwischen der proximalen Hülse und dem proximalen Ende des Rotors ermöglicht. Vorteilhafterweise greift am proximalen Ende des Rotors eine den Rotor im Betrieb drehende, innerhalb des Katheters geführte Rotorwelle an.

Das proximale Trageteil kann dabei eine Außenbuchse und einen in der Außenbuchse angeordneten Ring aufweisen, welcher auf dem proximalen Ende des Rotors verschiebbar gelagert ist. Beim aufeinander zu bewegen der beiden Hülsen gleitet dann der Ring des proximalen Trageteils auf dem Rotor.

Weitere Einzelheiten und vorteilhafte Ausgestaltungen der Erfindung sind der nachfolgenden Beschreibung zu entnehmen, anhand derer ein Ausführungsbeispiel der Erfindung näher beschrieben und erläutert ist.

Es zeigen:
Figur 1 eine perspektivische Ansicht auf einen Pumpenkopf einer erfindungsgemäßen Katheterpumpe mit teilaufgeschnittenen Hülsen des Käfigs;
Figur 2 eine vergrößerte Ansicht der teilaufgeschnittenen proximalen Hülse des Käfigs;
Figur 3 die teilaufgeschnittene distale Hülse des Käfigs; und
Figur 4 Längsschnitte durch die distale und proximale Hülse des Käfigs.

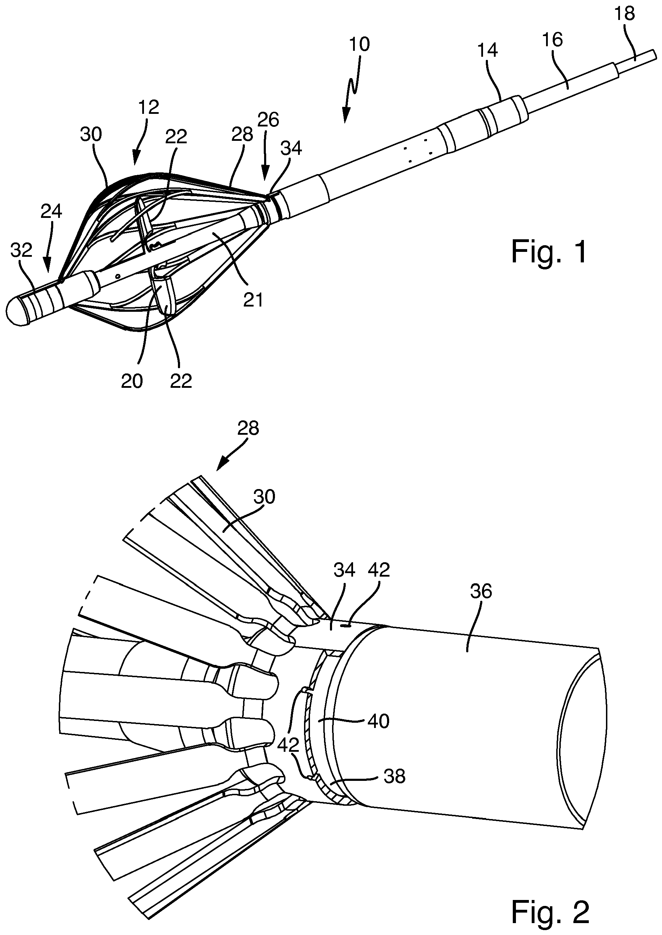

Die in der Figur 1 gezeigte Katheterpumpe 10 weist einen Pumpenkopf 12 auf, der in die Aorta oder das Herz eines Patienten eingeführt werden kann. Die Pumpe 10 umfasst einen Außenkatheter 14, einen Innenkatheter 16 und eine im Innenkatheter 16 verdrehbar angeordnete Rotorwelle 18. Über die Rotorwelle 18 kann ein in Figur 1 expandiertes Förderelement 20 in Form eines Rotors 21 mit ausgeklappten Propellern 22 angetrieben werden. Die Propeller 22 sind dabei zwischen einer distalen Lagerstelle 24 und einer proximalen Lagerstelle 26 angeordnet. Das Förderelement 20 bzw. die Propeller 22 werden von einem Käfig 28 umgeben, der zwei Hülsen 32 und 34 sowie zwischen den Hülsen 32 und 34 verlaufende Filamente 30 vorsieht. Im expandierten Zustand, der in Figur 1 gezeigt ist, ist der Käfig 28 bauchartig ausgebildet, so dass sich die Propeller 22 innerhalb des Käfigs 28 frei drehen können. Zum Einführen des Pumpenkopfes 12 in die Aorta oder das Herz ist der Pumpenkopf 12 nicht expandiert, sondern in einem kollabierten bzw. eingeklappten Zustand. In diesem kollabierten Zustand liegen die Propeller 22 nahe an der Drehachse des Rotors 21 und die Filamente 30 des Käfigs 18 befinden sich in einer parallel zur Drehachse des Rotors 21 verlaufenden Position.

Zum Expandieren des Förderelements 20 wird die proximale Hülse 34 in axialer Richtung hin zur distalen Hülse 32 bewegt, wodurch zunächst der Käfig 28 in die in Figur 1 gezeigte bauchige Lage verlagert wird und daran anschließend die Propeller 22 in die in der Figur 1 gezeigte ausgeklappte Lage expandieren. Das Bewegen der proximalen Hülse 34 relativ zur distalen Hülse 32 erfolgt durch eine axiale Bewegung des Außenkatheters 14 relativ zum Innenkatheter 16.

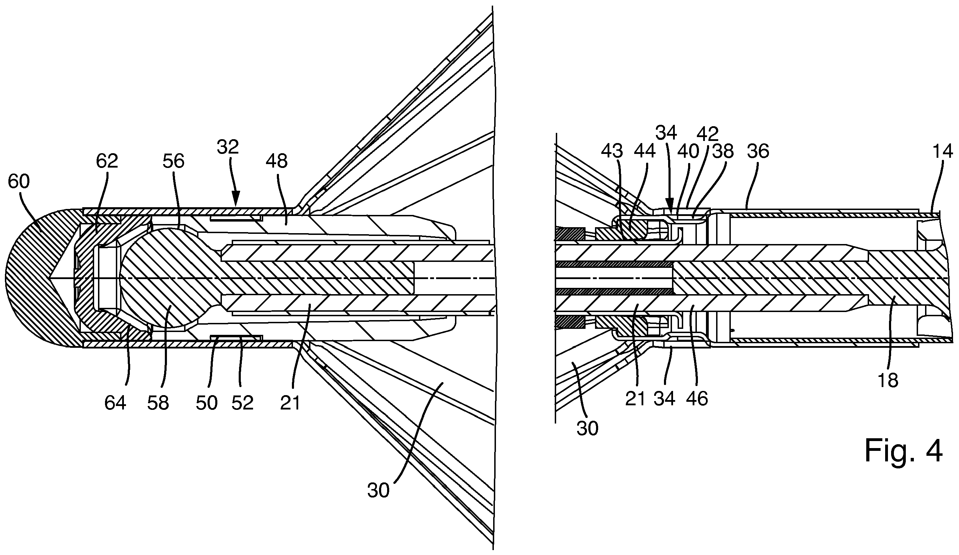

Wie aus Figur 2 deutlich wird, ist die distale Hülse 34 des Käfigs 28 an einem Trageteil 36 über ein Ringelement 38 angeordnet. Das Ringelement 38 befindet sich dabei in einer am Trageteil 36 vorgesehenen Umfangsnut 40, die im Schnitt gemäß Figur 4 deutlich zu erkennen ist. Das Ringelement 38 ist in der Umfangsnut 40 in axialer Richtung gefangen und insbesondere in axialer Richtung spielfrei oder wenigstens weitgehend spielfrei angeordnet. Zur Verbindung der Hülse 34 mit dem Ringelement 38 sieht die Hülse 34 langlochartige Ausnehmungen 42 vor, über welche die Hülse 34 mit dem Ringelement 38 verschweißt ist. Der Käfig 28 bzw. das Ringelement 38 ist dabei aus dem gleichen Material wie das Ringelement 38, vorzugsweise aus einer Nickel-Titan-Legierung. Dadurch, dass beide Bauteile aus dem gleichen Material bestehen, können diese Bauteile in den Ausnehmungen 42 vergleichsweise einfach miteinander verschweißt werden. Dadurch, dass das Ringelement 38 in der Umfangsnut 40 formschlüssig gefangen angeordnet ist, kann eine axiale Bewegungskopplung der Hülse 34 mit dem Trageteil 36 realisiert werden. Das Trageteil 36 ist dabei vorzugsweise aus einer Edelstahllegierung.

Wie aus Figur 4 deutlich wird, ist das proximale Trageteil 36 als Außenbuchse mit einem in der Außenbuchse angeordneten Ring 44 gebildet, welcher auf dem proximalen Endabschnitt des Rotors 21 verschiebbar gelagert angeordnet ist. Der Ring bildet dabei also eine Schiebelageraufnahme 43. Zum Kollabieren bzw. Einklappen des Käfigs 28 wird die proximale Hülse 34 relativ zum Rotor 21 in die der distalen Hülse 32 abgewandte Richtung bewegt, wobei dann das Ringelement 44 auf dem proximalen Bereich 46 des Rotors 21 schiebegelagert geführt wird. Das Verschieben des proximalen Trageteils 36 auf dem Rotor 21 erfolgt dabei durch Bewegen des Außenkatheters 14 relativ zum mit dem Rotor 21 in axialer Richtung bewegungsgekoppelten Innenkatheter 16.

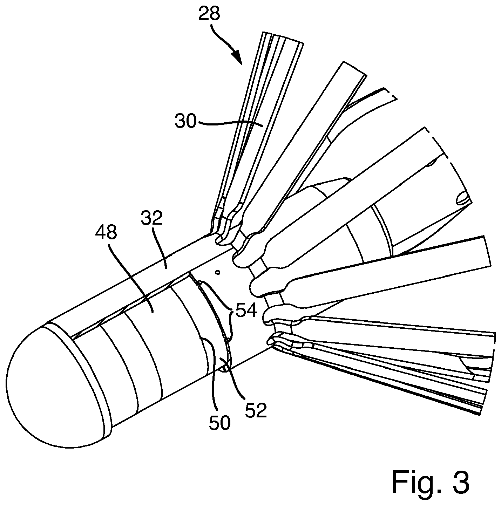

Aus der Figur 3 wird deutlich, dass die distale Hülse 32 entsprechend der proximalen Hülse 34 ein distales Trageteil 48 umgibt, welches eine Umfangsnut 50 vorsieht, in der in axialer Richtung ein Ringelement 52 spielfrei gelagert bzw. gefangen angeordnet ist. Die distale Hülse 32 sieht entsprechend der proximalen Hülse 34 Ausnehmungen 54 vor, in welchen sich Schweißpunkte befinden, mittels denen das Ringelement 52 mit der distalen Hülse 32 insbesondere punktverschweißt ist.

In Figur 4, in der das distale Trageteil 48 im Längsschnitt gezeigt ist, ist die Umfangsnut 50 sowie das in der Umfangsnut 50 vorgesehene Ringelement 52 deutlich zu erkennen. Das distale Trageteil 48 weist dabei eine Drehlageraufnahme 56 für den am distalen Ende des Rotors 21 vorgesehenen Kugelkopf 58 auf. Am distalen Ende ist eine Kappe 60 vorgesehen, welche das offene Ende der Hülse 32 abdeckt. Zwischen der Kappe 60 und dem Trageteil 48 ist ein Lagerdeckel 62 vorgesehen, der einen Aufnahmeabschnitt 64 für den Kugelkopf 58 vorsieht. Der Kugelkopf 58 ist folglich sicher zwischen dem Trageteil 48 und dem Lagerdeckel 62 drehgelagert angeordnet.

Über die Schweißverbindung der Hülsen 32 und 34 mit den zugehörigen Ringelementen 38 und 52 und über die in den Trageteilen 36 und 48 vorgesehenen Umfangsnuten 40 und 50, in denen die Ringelemente 38 und 52 formschlüssig angeordnet sind, kann eine funktionssichere Bewegungskopplung und Kraftübertragung in axialer Richtung zwischen den Hülsen 32 und 34 sowie den Trageteilen 36 und 48 realisiert werden, obwohl der Käfig 28 samt Hülsen 32 und 34 aus einem anderen Material als die Trageteile 36 und 48 sind.

## Patentansprüche

1. Katheterpumpe (10) mit einem Pumpenkopf (12) zum Einsetzen in das arterielle Gefäßsystem, wobei der Pumpenkopf (12) ein Förderelement (20) aufweist, das aus einer eingeklappten Einführlage, in der der Pumpenkopf (12) in das arterielle Gefäßsystem einführbar ist, in eine ausgeklappte Betriebslage verlagerbar ist, und einen das Förderelement (20) umgebenden Käfig (28) aufweist, wobei der Käfig (28) eine distale und eine proximale Hülse (32, 34) sowie zwischen den Hülsen verlaufende Filamente (30) aufweist, **dadurch gekennzeichnet, dass** im Bereich der distalen und/oder der proximalen Hülse (32, 34) ein mit der jeweiligen Hülse (32, 34) in axialer Richtung gekoppeltes Trageteil (36, 48) vorgesehen ist, wobei das Trageteil (36, 48) eine Umfangsnut (40, 50) aufweist, in der ein in axialer Richtung gefangenes Ringelelement (38, 52) vorgesehen ist, und wobei die Hülse (32, 34) wenigstens eine Ausnehmung (42, 54) aufweist, in welcher die Hülse (32, 34) mit dem Ringelement (38, 52) verschweißt ist.

2. Katheterpumpe (10) nach Anspruch 1, **dadurch gekennzeichnet, dass** der Käfig (28) samt Hülsen (32, 34) sowie das jeweilige Ringelement (38, 52) aus dem gleichen Material und vorzugsweise aus einer Formgedächtnislegierung sind.

3. Katheterpumpe (10) nach Anspruch 1 oder 2, **dadurch gekennzeichnet, dass** die Trageteile (36, 48) aus einem zum Käfig (28) unterschiedlichen Material und vorzugsweise aus einer Edelstahllegierung sind.

4. Katheterpumpe (10) nach Anspruch 1, 2 oder 3, **dadurch gekennzeichnet, dass** die jeweilige Hülse (32, 34) mehrere Ausnehmungen (42, 54) aufweist, über welche das Ringelement (38, 52) mit den Hülsen (32, 34) verschweißt ist.

5. Katheterpumpe (10) nach einem der vorhergehenden Ansprüche, **dadurch gekennzeichnet, dass** die distale Hülse (32) an einem distalen Trageteil (48) angeordnet ist, wobei das distale Trageteil (48) eine Drehlageraufnahme (56) für das distale Ende (58) des Rotors (21) aufweist.

6. Katheterpumpe (10) nach einem der vorhergehenden Ansprüche, **dadurch gekennzeichnet, dass** die proximale Hülse (34) an einem proximalen Trageteil (36) angeordnet ist, wobei das proximale Trageteil (36) eine Schiebelageraufnahme (43) für den proximalen Abschnitt des Rotors (21) aufweist.

7. Katheterpumpe (10) nach Anspruch 6, **dadurch gekennzeichnet, dass** das proximale Trageteil (36) eine Außenbuchse und einen in der Außenbuchse angeordneten Ring (44) aufweist, in dem der proximale Abschnitt des Rotors (21) verschiebbar gelagert ist.

## Claims

1. Catheter pump (10) with a pump head (12) for insertion into the arterial vascular system, whereby the pump head (12) has a conveyor element (20) that can transition from a folded-up insertion state in which the pump head (12) can be inserted into the arterial vascular system, to an unfolded operating state, and with a cage (28) encompassing the conveyor element (20), whereby the cage (28) has a distal and proximal sleeve (32, 34) as well as filaments (30) between the sleeves, distinguished by the fact that there is a bearing component (36, 48) axially coupled with the respective sleeve (32, 34) near the distal and/or proximal sleeve (32, 34), whereby the bearing component (36, 48) has a circumferential groove (40, 50) in which an axially interlocked ring element (38, 52) is located, and whereby the sleeve (32, 34) has at least one recess (42, 54) in which the sleeve (32, 34) is welded with the ring element (38, 52).

2. Catheter pump (10) as per claim 1, distinguished by the fact that the cage (28) and sleeves (32, 34), as well as the respective ring element (38, 52), are made from the same material, preferably a smart memory alloy.

3. Catheter pump (10) as per claim 1 or 2, distinguished by the fact that the bearing components (36, 48) are made from a different material than the cage (28), preferably a stainless steel alloy.

4. Catheter pump (10) as per claim 1, 2 or 3, distinguished by the fact that the respective sleeve (32, 34) has multiple recesses (42, 54) via which the ring element (38, 52) is welded with the sleeves (32, 34) .

5. Catheter pump (10) as per one of the preceding claims, distinguished by the fact that the distal sleeve (32) is attached to a distal bearing component (48), whereby the distal bearing component (48) has a pivot bearing socket (56) for the distal end (58) of the rotor (21).

6. Catheter pump (10) as per one of the preceding claims, distinguished by the fact that the proximal sleeve (34) is attached to a proximal bearing component (36), whereby the proximal bearing component (36) has a slide bearing socket (43) for the proximal section of the rotor (21).

7. Catheter pump (10) as per claim 6, distinguished by the fact that the proximal bearing component (36) has an outer bushing and a ring (44) within the outer bushing, in which the proximal section of the rotor (21) is located with a slide bearing.

## Revendications

1. Pompe à cathéter (10) pourvue d'une tête de pompe (12) destinée à être insérée dans le système vasculaire artériel, dans laquelle la tête de pompe (12) présente un élément de refoulement (20), qui peut être déplacé d'une position d'introduction repliée, dans laquelle la tête de pompe (12) peut être introduite dans le système vasculaire artériel, dans une position de fonctionnement dépliée, et une cage (28) entourant l'élément de refoulement (20), dans laquelle la cage (28) présente un manchon distal et un manchon proximal (32, 34) ainsi que des filaments (30) s'étendant entre les manchons, **caractérisée en ce qu'**une pièce de support (36, 48) accouplée au manchon (32, 34) respectif dans la direction axiale est prévue dans la zone du manchon distal et/ou proximal (32, 34), dans laquelle la pièce de support (36, 48) présente une rainure périphérique (40, 50), dans laquelle un élément annulaire (38, 52) capturé dans la direction axiale est prévu, et dans laquelle le manchon (32, 34) présente au moins un évidement (42, 54), dans lequel le manchon (32, 34) est soudé à l'élément annulaire (38, 52).

2. Pompe à cathéter (10) selon la revendication 1, **caractérisée en ce que** la cage (28) y compris les manchons (32, 34) ainsi que l'élément annulaire (38, 52) respectif sont composés du même matériau et de préférence d'un alliage à mémoire de forme.

3. Pompe à cathéter (10) selon la revendication 1 ou 2, **caractérisée en ce que** les pièces de support (36, 48) sont composées d'un matériau différent de la cage (28) et de préférence d'un alliage d'acier inoxydable.

4. Pompe à cathéter (10) selon la revendication 1, 2 ou 3, **caractérisée en ce que** le manchon (32, 34) respectif présente plusieurs évidements (42, 54), par l'intermédiaire desquels l'élément annulaire (38, 52) est soudé aux manchons (32, 34).

5. Pompe à cathéter (10) selon l'une quelconque des revendications précédentes, **caractérisée en ce que** le manchon distal (32) est disposé sur une pièce de support (48) distale, dans laquelle la pièce de support (48) distale présente un logement de palier rotatif (56) pour l'extrémité distale (58) du rotor (21).

6. Pompe à cathéter (10) selon l'une quelconque des revendications précédentes, **caractérisée en ce que** le manchon proximal (34) est disposé sur une pièce de support (36) proximale, dans laquelle la pièce de support (36) proximale présente un logement de palier coulissant (43) pour la section proximale du rotor (21).

7. Pompe à cathéter (10) selon la revendication 6, **caractérisée en ce que** la pièce de support (36) proximale présente un manchon extérieur et une bague (44), disposée dans le manchon extérieur, dans laquelle la section proximale du rotor (21) est montée coulissante.
